# EUROPEAN PATENT APPLICATION

(11) **EP 1 382 364 A1**
(43) Date of publication of application: **21.01.2004**
(21) Application number: 03015161.7
(22) Date of filing: 04.07.2003
(51) Int. Cl.: A61M 16/20, A61M 16/00, A61M 16/12

(54) **Intake valve**

(30) Priority: 15.07.2002 NO 20023404
(71) Applicant: Laerdal Medical AS, 4002 Stavanger (NO)
(72) Inventor: Rafoss, Ivan, 4324 Sandnes (NO)
(74) Representative: Wolff, Felix, Dr.

(57) **Abstract**

Reusable intake valve for a resuscitator bag, comprising an inner part (31), an outer part (32) and a valve housing (39). The inner (31) and outer (32) parts are separate parts designed to be connected at an opening (6) in the bag (1) and retain the edge of the opening (6) between them. The inner part is equipped with a first valve (37) designed to admit air into the bag (1) and close for air flow in the opposite direction. The valve housing is equipped with a second and a third valve (40, 41), where the second valve (40) is designed to admit air into the valve housing (39) from the surroundings and close for air flow in the opposite direction. The third valve (41) is designed to release air from the valve housing (39) to the surroundings and close for air flow in the opposite direction. The valve housing has a pipe stub (42) for connection to an oxygen reservoir (3) and a pipe stub (44) for supply of oxygen. The outer part (32) and the valve housing (39) are integrated into one piece.

## Description

A resuscitator bag is used with a patient mask or an endotracheal tube to deliver air to and ventilate a patient with a reduced or absent respiration. The equipment is frequently used by medical personnel in ambulances, hospitals and casualty wards.

In principle, the equipment can consist of the actual bag, which is a soft bag made of e.g. silicon or PVC, a patient valve provided on the bag and which controls the air to and from the patient, a mask or alternatively an endotracheal tube that leads the air into and out of the patients respiratory passages, and intake valve that admits air into the bag, and an oxygen reservoir, as well as an oxygen hose for delivery of oxygen to the bag. The delivery of oxygen to the oxygen reservoir takes place through an additional valve housing, making it possible to deliver a higher oxygen concentration to the patient.

Today's intake valves have remained relatively unchanged w.r.t. form and function since the 1960s. These intake valves generally consist of an inner part and an outer part and a diaphragm valve that allows air to be sucked into the bag when the bag expands. The same valve prevents air from flowing back out the same way, by the diaphragm closing the outlet in the opposite direction. Therefore, when the operator compresses the bag, air is forced out of the bag and to the patient.

With time, it has become more common to feed the patient extra oxygen to give better treatment results. For that reason, it is known that the outer part of the intake valve may be constructed with two socket pieces, one for connection of oxygen and one for connection of an additional valve housing. This valve housing has several functions related to the delivery of oxygen to the patient:
1. Connection point for an oxygen reservoir that collects oxygen between every time the operator compresses the bag. With this, the bag can suck oxygen in from the reservoir every time the bag expands, and when this is used correctly, the patient may receive almost 100% oxygen instead of 21% oxygen, as is normal in atmospheric air. Oxygen from an oxygen tube connected to the outer part will fill the reservoir with a constant but adjustable flow of oxygen.
2. In the event of failure in the oxygen supply or the reservoir, a diaphragm valve will allow atmospheric air to be sucked into the valve housing and into the bag.
3. In the case of excessive admission of oxygen, relative to that required for the 'bagging" (the repeated compression and inflation of the bag) and the volume of the reservoir, excess oxygen will be released through another diaphragm valve.

Today's solution entails keeping a separate product in stock, which product the operator must place on the intake valve in order to obtain the desired function of providing supplemental oxygen to the patient during bagging.

In a known design belonging to the Applicant, the two products, the intake valve and the extra valve housing, contain a total of nine different components, of which 8 are non-identical. This leads to high production and assembly costs and involves a labour-intensive process for the operator who reuses the products and needs to maintain them, among other things by completely disassembling them for cleaning and any required sterilisation.

An intake valve with an integrated oxygen supply is also known, from US 5163424. However, this valve is designed to be disposable, together with the bag, patient valve and oxygen reservoir. I.e. the parts are not intended to be disassembled for cleaning. The valve housing is connected to the bag through the edge of the bag opening being placed in a groove in the valve housing. In order to achieve this, the bag opening must be expanded until it can be slipped into the groove. To prevent air from escaping between the bag and the valve housing, and prevent the bag from slipping out of the groove when the bag is compressed, the edge of the bag must abut the valve housing with a certain amount of force. Consequently, special tools are likely to be required in order to get the edge of the opening into the groove. And therefore, getting the edge of the opening out of the groove again is not going to be very easy. In the commercially available disposable product, the transition between the bag and the valve housing is stapled together to prevent it from detaching. It goes without saying that this connection can not be undone and reformed without damaging the product.

Furthermore, the oxygen reservoir is held in place by means of a sheath that is pulled over the valve housing. The sheath is provided with a bead that slips over an edge on the valve housing. It is probably not possible to remove the sheath again without damaging it or the valve housing. These two factors alone make this equipment totally unsuited for multiple use.

The present invention aims to provide a new, reusable intake valve that integrates said functions of the known multi-use intake valve and the additional valve housing.

The invention also aims to significantly reduce the number of components that is required for the product to function. According to a presently preferred embodiment, the total number of components has been reduced from nine to five, and the number of non-identical components has been reduced from eight to three. The preferred embodiment may also include the use of an additional component to protect the valve against external influences. This makes it easier to use, thereby providing increased patient safety.

The present invention may also provide a number of additional benefits:
- The same type of diaphragm valve can be used for three different valve functions while at the same time meeting the existing requirements for flow resistance. This simplifies the manufacturing process and maintenance of the product, thereby saving costs. Moreover, it results in the product having two spare valves. If the suction valve were to be damaged, a life-threatening situation may still be avoided by changing the valves over.
- The diaphragm valves and the outer and inner parts can be designed to prevent or make it impossible not to detect, through prescribed testing, incorrect assembly (which may be life-threatening).
- The construction of the integrated intake valve with two diaphragm valves may be realised by mounting the valves on the side faces of the outer part. This allows the use of larger types of diaphragm valves on an integrated intake valve than that which is possible with valves mounted in the same plane across the longitudinal axis of the bag. The larger diameter diaphragm valves can be pretensioned and constructed in a stiffer material, so as not to leak. At the same time, the large diameter gives a low flow resistance. This ensures good performance.
- The new outer part can be designed so as to function even if it is installed on an old inner part by mistake, and conversely, a new inner part will function even if it is installed on an old outer part by mistake.

The invention will now be explained in greater detail, through explaining the construction and principle of operation of a known intake valve and by explaining an embodiment of the present invention, where:
Figure 1 shows a bag with a patient valve, an oxygen reservoir and an intake valve with an additional valve housing of a known type;
Figure 2 is an exploded view of the known intake valve with an additional valve housing in Figure 1;
Figure 3 shows a section through the known intake valve with an additional valve housing;
Figure 4 shows a bag with a patient valve, an oxygen reservoir and an intake valve according to the present invention;
Figure 5 is an exploded view of the intake valve according to the present invention; and
Figure 6 shows a section through the intake valve according to the present invention.

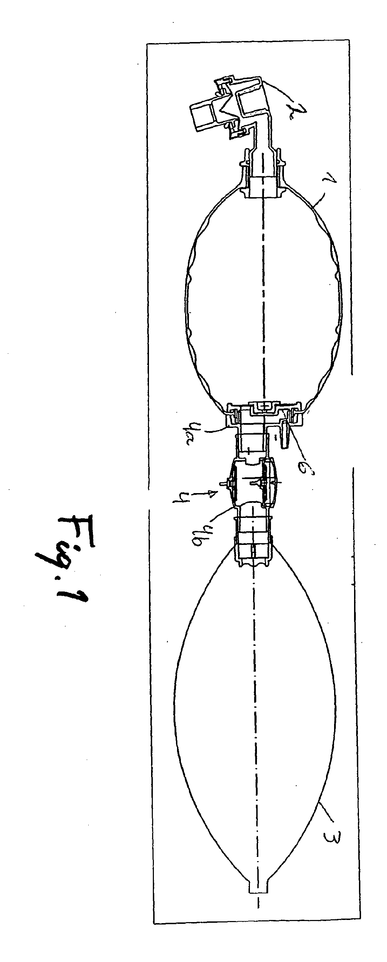

Figure 1 shows a bag 1 with a patient valve 2, an oxygen reservoir 3 and an intake valve 4 according to prior art. The intake valve 4 consists of the actual intake valve 4a and an additional valve housing 4b. The actual intake valve is installed in a first opening 6 in the bag 1.

In operation, the oxygen reservoir 3 is filled from an oxygen source (not shown) via an oxygen connection 15 on the intake valve 4a. The oxygen flows into the housing of the intake valve 4a at a constant pressure, and via the additional valve housing 4b into the oxygen reservoir 3 in order to fill this.

When the operator compresses the bag 1, oxygen is forced out of the bag 1 through an opening 7 at the opposite end of the bag, and through the patient valve to the patient. When the operator releases the grip on the bag 1 to allow it to resume its ball/almond shape, air/oxygen is sucked into the bag 1 from the oxygen reservoir 3. This oxygen flows via the additional valve housing 4b and the actual intake valve 4a into the bag 1.

The patient valve 2 is constructed so as to allow air from the bag in to the patient. Exhaled air from the patient is prevented from flowing back to the bag, and is passed out to the surroundings. The patient valve will not be explained in greater detail, as its construction and operation is the same for the present invention as for prior art.

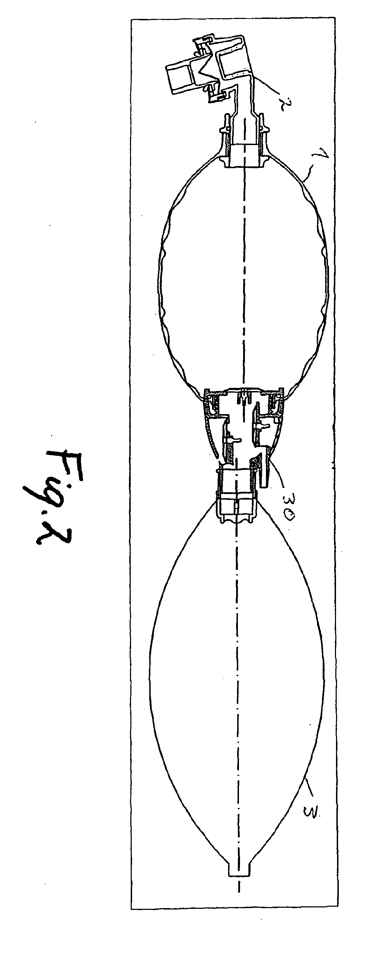

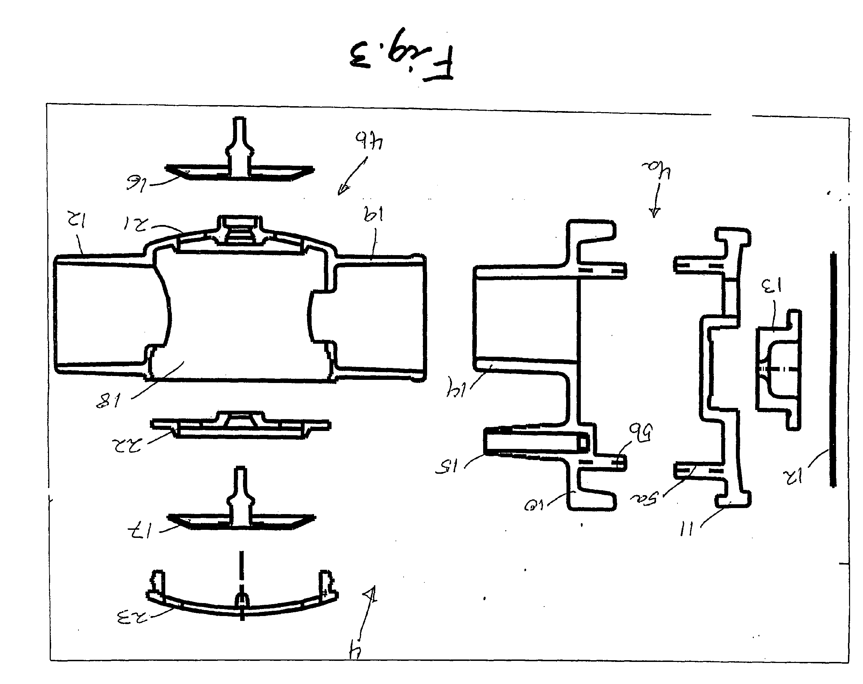

Figure 2 is an exploded view of the known intake valve 4, and Figure 3 shows the valve in the assembled state. The actual intake valve 4a consists of an outer part 10, an inner part 11, a diaphragm 12 and a diaphragm holder 13. The inner 11 and outer 10 parts are equipped with complementary threads 5a and 5b to allow these to be screwed together. The inner part 11 is placed on the inside of the bag 1, and the outer part 10 on the outside of the bag 1, so that when these parts are screwed together at the bag opening 6, the bag will be pinned between the parts, thus forming an airtight and secure connection between the intake valve 4a and the bag 1. The diaphragm 12 and the diaphragm holder 13 are fitted in the inner part 11 by the diaphragm holder 13 being passed through a hole in the diaphragm 12 and on to a screw connection in the inner part 11. This fixes the middle section of the diaphragm 12, while the peripheral parts of the diaphragm 12 only rest against the inner part 11. In the case of an overpressure in the intake valve 4 relative to the bag 1, the peripheral parts of the diaphragm 12 will thereby be lifted away from their position of abutment against the inner part 11, allowing air to pass by the diaphragm 12. In the case of overpressure on the opposite side of the diaphragm 12, this will be forced against the inner part 11 and not allow air to pass.

The outer part has a large diameter pipe stub 14. The additional valve housing 4b is connected to the large diameter pipe stub 14 and used to deliver air with a higher concentration of oxygen than that of the ambient air (alternatively 100% oxygen) to the patient. The valve housing 4b has a middle section 18 and two pipe stubs 19, 20 facing in opposite directions from the middle section 18. Two diaphragm valves 16 and 17 (so-called mushroom valves) are provided at the middle section 18. The valve 16 is placed with the diaphragm on the inside of a wall 21 in the middle section 18, so as to open for air from the surroundings to flow into the intake valve, and close for air in the opposite direction. The valve 17 is positioned with the diaphragm on the outside of a separate wall 22, which is attached to the middle section so as to open for air from the valve housing 4b to flow out to the surroundings. Outside the valve 17 is a protective cover 23 to prevent objects (fingers) from blocking the valve function. Air may pass by the cover 23.

A pipe stub 24 is attached to the oxygen reservoir 3, which pipe stub may be pulled onto the additional valve housing 4b.

The actual intake valve 4a is also equipped with the above oxygen connection 15. This has a smaller diameter that the pipe stub 14. Oxygen is supplied through this from an oxygen source (not shown).

When the bag is compressed, the diaphragm 12 will, as a result of the increased pressure, press against the inner part 11 and prevent air from flowing from the bag and out into the intake valve 4a. Oxygen will flow in via the oxygen connection 15, on through the additional valve housing 4b and into the oxygen reservoir 3. If the oxygen reservoir 3 is completely filled before the bag 1 is released, the valve 17 will open in order to release the excess oxygen. The valve 17 is pretensioned to open at a set pressure.

If, for some reason, the oxygen supply fails and the flow of oxygen is reduced or ceases completely, the valve 16 will open when the bag 1 is released and so creates an underpressure in the bag 1. By so doing, ambient air will flow into the bag 1. The patient then receives normal air with at least 21% oxygen.

The invention will now be explained in greater detail with reference to an embodiment shown in Figures 2, 5 and 6.

As shown in Figure 2, the equipment generally consists of a bag 1, a patient valve 2, an oxygen reservoir 3 and an intake valve 30 according to the invention. The bag 1, the patient valve 2 and the oxygen reservoir 3 may be identical to those shown in Figure 1, and as such these parts will not be explained in greater detail.

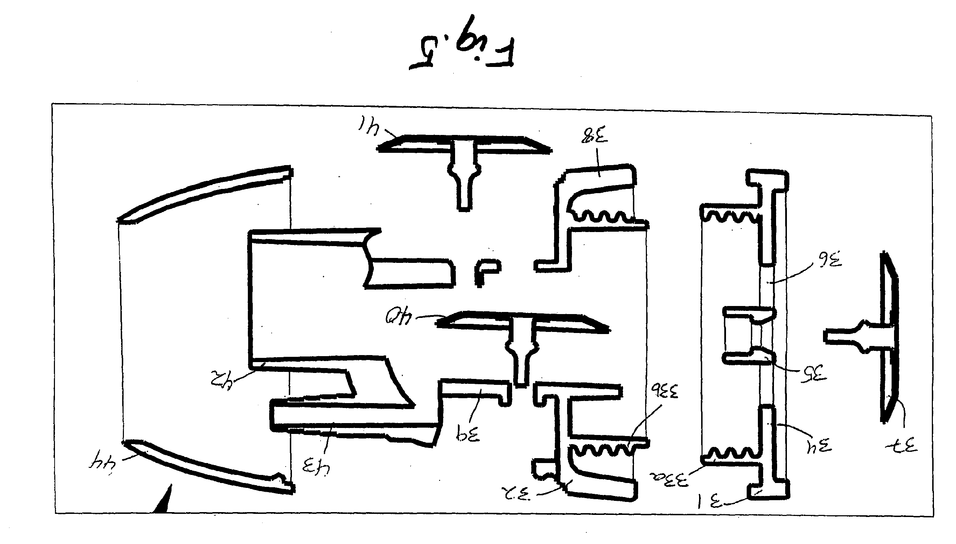

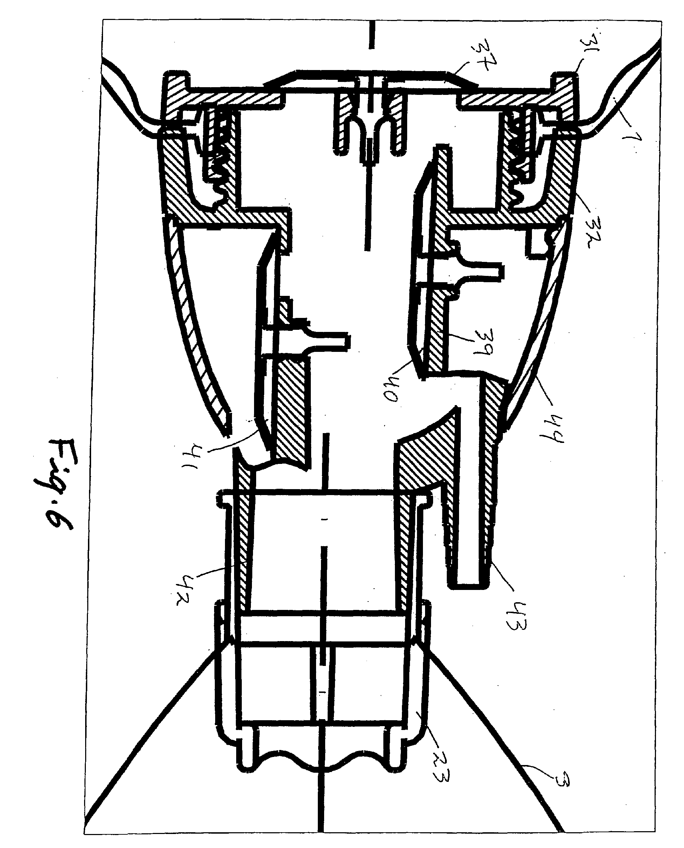

The intake valve according to the invention will now be explained in greater detail with reference to Figures 5 and 6, where Figure 5 is an exploded view and Figure 6 is a longitudinal section. Please note here that the longitudinal section is not taken along one plane, but that the left hand and right hand parts of the figure are sections along two respective planes located at right angles to each other.

The intake valve 30 according to the invention also has an inner part 31 and an outer part 32. The inner and outer parts have complementary threads 33a and 33b that, in the same way as for the known intake valve 4a, pins the bag between them when the parts are screwed together.

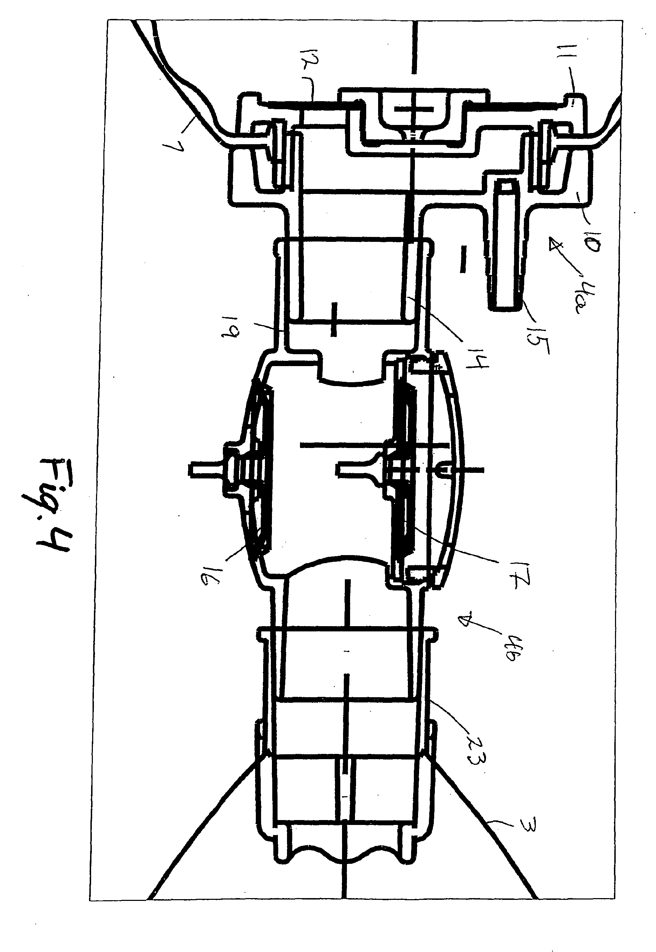

The inner part 31 comprises a disc 34 from which the threaded portion 33a projects. Furthermore, a sleeve portion 35 is formed centrally in the disc. Around the sleeve portion 35 is a number of holes 36. The stem of a mushroom valve 37, which in principle is designed the same way as valves 16 and 17 in Figures 3 and 4, is pressed into the sleeve portion 35. Here, the valve stem is fixed by a snap fit. The snap fit is formed so as to allow the valve 37 to be removed by pressing against the valve stem or pulling the diaphragm.

The outer part 32 comprises a cup-shaped portion 38 extending outside the threaded portion 33b, and which in the coupled-up state pins the bag 1 between itself and the inner part 31. A valve housing 39 projects centrally in the cup-shaped portion, which valve housing corresponds to the valve housing 4b in Figures 3 and 4. Two mushroom valves 40 and 41 are provided in the valve housing 39. Preferably, the valves 40 and 41 are identical to the valve 37, so as to make the valves interchangeable and make it simpler to keep these in stock. The valve 40 is arranged with the diaphragm inside the housing 39, to make it open up to external pressure, while the valve 41 is arranged on the outside of the housing 39, to make it open up to internal pressure. The valve 41 is pretensioned to open at a set pressure, in the same manner as the valve 17 in Figures 3 and 4. This is achieved in a manner that is known per se, by the valve 41 having to be pushed against the valve housing with a certain amount of force before it snaps into place.

A pipe stub 42 projects from the valve housing 39, which pipe stub is matched to the pipe stub 23 on the oxygen reservoir. Projecting from the valve housing 39 is also an oxygen connection 43, for connection of an oxygen hose (not shown). In reality, the oxygen connection projects from the valve housing 39 on the side facing out of the plane of the paper. However, it has been drawn in the plane of the paper in order to render it visible.

A protective sheath 44 may be guided onto the outside of the valve housing 39 and brought into abutment (e.g. a snap fit) against the outer part 32. The sheath 44 extends from the pipe stubs 42 and 43 to the outer part 32. The sheath 44 is permeable to air.

The principle of operation of the intake valve according to the present invention is in principle the same as for the known intake valve and the additional valve housing according to Figures 1, 3 and 4. Oxygen flows in through oxygen connection 43 and into the oxygen reservoir 3. When the oxygen reservoir is full, the valve 41 opens to release excess oxygen.

When the bag 1 is compressed, the valve 37 closes, and air is forced out through the patient valve 2 to the patient.

If the oxygen supply fails, the valve 40 will open as soon as the bag 1 is released. This will result in the bag drawing air from the surroundings through the valve 40.

Preferably, the diaphragms of the valves 37, 40, 41 are identical and made from a silicon material, so as to make them interchangeable. Preferably, the outer part 32 with the valve housing 39 is made from an inflexible transparent plastic material. The remaining parts of the valve 30 may also be made from the same inflexible plastic material.

Preferably, the outer part according to the invention is constructed so as to allow it to be connected to the inner part of an intake valve of an existing type such as described in connection with Figures 1, 3 and 4, which is marketed as Laerdal Silicone Resuscitator, under article number 51 04 00. This effectively eliminates the risk of a coupling of old and new parts having any consequences for the operation.

Thus the present invention provides a reusable intake valve that consists of very few parts, is easy to assemble and limits the scope for incorrect interconnection.

## Claims

1. Reusable intake valve for a resuscitator bag, comprising an inner part (31), an outer part (32) and a valve housing (39), where the inner (31) and outer (32) parts are separate parts designed to be connected at an opening (6) in the bag (1) and retain the edge of the opening (6) between them, the inner part being equipped with a first valve (37) designed to admit air into the bag (1) and close for air flow in the opposite direction, where the valve housing is equipped with a second and a third valve (40, 41), where the second valve (40) is designed to admit air into the valve housing (39) from the surroundings and close for air flow in the opposite direction and the third valve (41) is designed to release air from the valve housing (39) to the surroundings and close for air flow in the opposite direction, and where the valve housing has a pipe stub (42) for connection to an oxygen reservoir (3) and that the intake valve has a pipe stub (44) for supply of oxygen, **characterised in that** the outer part (32) and the valve housing (39) are integrated into one piece.

2. An intake valve according to Claim 2, **characterised in that** the pipe stub (43) for supply of oxygen is disposed on the valve housing (39).

3. An intake valve according to one of the preceding claims, **characterised in that** the first, second and third valves (37, 40, 41) are identical and interchangeable.

4. An intake valve according to Claim 3, **characterised in that** the valves (37, 40, 41) comprise a diaphragm and a stem attached to the diaphragm, where the stem is designed to be passed through a hole in the intake valve for a snap fit with the intake valve.

5. An intake valve according to Claim 4, **characterised in that** the stem of the second valve (40) is passed from the outside and through a side wall in the valve housing (39), and the stem of the third valve (41) is passed from the inside of the valve housing (39) and through a side wall of the valve housing (39).

6. An intake valve according to one of the preceding claims, **characterised in that** the inner part (31) has athreaded portion (33a) designed to be screwed together with a complementary threaded portion (33b) on the outer part (32).

7. An intake valve according to Claim 6, **characterised in that** the inner part (31) is constructed to allow it to be connected to an outer part of the existing type marketed as Laerdal Silicone Resuscitator, by Laerdal Medical AS, and that the outer part (32) is constructed in a corresponding manner, to allow it to be connected to an inner part of the existing type marketed as Laerdal Silicone Resuscitator, by Laerdal Medical AS

8. An intake valve according to one of the preceding claims, **characterised in that** it comprises a sheath (44) designed to be pulled onto the outside of the valve housing (39) to cover at least an area between the outer part (32) and the piping stub (42), in order to protect the valve housing (39) and the valves (40, 41) against external influences.
